# EUROPEAN PATENT APPLICATION

(11) **EP 1 635 295 A1**
(43) Date of publication of application: **15.03.2006**
(21) Application number: 05251836.2
(22) Date of filing: 24.03.2005
(51) Int. Cl.: G06T 11/00, G06T 7/00, A61B 6/00

(54) **User interface for CT scan analysis**

(30) Priority: 10.09.2004 GB 0420147
(71) Applicant: Medicsight PLC, London W1J 5AT (GB)
(72) Inventor: Dehmeshki, Jamshid, Dr., London (GB)
(74) Representative: Cross, James Peter Archibald

(57) **Abstract**

An original scan image and an enhanced scan image derived from the original scan image are displayed side by side or alternately, to facilitate comparison between the original and enhanced image. The user is able to change enhancement parameters of the enhanced image while viewing the original and enhanced image, to obtain the most suitable settings for the image while observing the effect on the enhanced image and comparing this with his or her own analysis of the original image. The adjusted parameters may then be applied to other parts of the original image so as to provide a more accurate analysis of those parts.

## Description

### Field of the Invention

The present invention relates to a user interface for CT scan analysis, and particularly for displaying a computed tomography (CT) scan image which is enhanced so as to highlight potential lesions or other abnormalities.

### Background of the Invention

In conventional analysis of CT images, a radiologist visually inspects each slice of a CT scan, using his or her expertise to identify abnormalities and to distinguish them from normal structures. The task can be made easier by storing the scan image on a computer and providing a user interface which allows the user to move rapidly between slices and visualise the structures in different ways. However, the process is time consuming and must be performed with great care in case any abnormalities are missed.

To replace some or all of the work of the radiologist, Computer Assisted Detection (CAD) software has been designed to analyse the scan image and detect potential lesions. The detection can be performed semi-automatically, with some interaction with the radiologist, or automatically, involving no interaction beyond the selection of the image to be analysed. For example, the applicant's MedicHeart™, MedicLung™ and MedicColon™ diagnostic software perform semiautomatic diagnosis of CT scans of the heart, lung and colon respectively.

In practice, the results of CAD must be checked by a radiologist as a safeguard. If the software is used as the 'first reader', the radiologist only verifies the results produced by the software and does not analyse the original CT scan. To be effective as a 'first reader', the software must have both high sensitivity (i.e. a low percentage of missed lesions) and high specificity (i.e. a low percentage of false positives), because the radiologist may make medically important decisions based on the results. 'First reader' has the greatest potential to save radiologists' time, but it is a great challenge to achieve both high sensitivity and high specificity.

Alternatively, the software can be used as a 'second reader', where the radiologist makes a preliminary diagnosis based entirely on the original images, and then runs the software as a check for any missed lesions. When used as a 'second reader', the software does not actually save time, but can assist the radiologist in making better diagnoses. The software does not need to have particularly high sensitivity or specificity, so long as it leads to more accurate diagnoses than an unassisted radiologist. Used in this way, the software is analogous to a spelling or grammar checker for word-processing - it merely draws the user's attention to oversights, rather than replacing the actions of the user.

It would be desirable to produce a user interface for analysis of CT scans that does not need to be as accurate as 'first reader' software but saves more time than 'second reader' software.

The document WO-A-03/077203 discloses a user interface which allows corresponding areas from different scans to be displayed side-by-side.

A further problem is that many CAD algorithms rely for detection on a predefined set of parameter ranges. For example the Agatston method, as originally described in 'Quantification of coronary artery calcium using ultrafast computed tomography', Agatston AS, Janowitz WR, Hildner FJ et al., J Am Coll Cardiol 1990 15:827-832, applies a threshold of 130 Hounsfield units (HU) to the CT image, and identifies all pixels above that threshold as containing calcium. A scoring system is then used to rate the severity of the calcification, based on the number of pixels above the threshold multiplied by a weight based on the highest intensity within the calcification. If the highest intensity is between 130 and 200 HU, then the weight is 1; if between 200 and 300 HU, the weight is 2; and if over 300 HU, the weight is 3. The values of the threshold and the weights are based on empirical studies of coronary scans and the subsequent outcome for the patients. However, there is continuing debate as to which parameter ranges give the most accurate results; different ranges may be appropriate for different scan images.

The document US6058322 discloses an interactive user modification function in which the software displays detected microcalcifications and the user may then add or delete microcalcifications. The software modifies its estimated likelihood of malignancy accordingly.

### Statement of the Invention

According to one aspect of the invention, there is provided a method of displaying a CT scan image, comprising displaying an original scan image and displaying an enhanced scan image derived from the original scan image. Preferably, the original and enhanced images are displayed with similar image attributes, such as size and scale, to facilitate comparison between the original and enhanced image. The enhanced image may be enhanced so as to identify to the user lesions or abnormalities in the original image.

In one embodiment, the original and enhanced images are displayed simultaneously. In another embodiment, the original and enhanced images are displayed alternately, with a similar size, scale and position. Preferably, the original and enhanced images are displayed on the same display device.

An advantage of these arrangements is that the original image can be visually checked against the enhanced image without the enhanced image obscuring features of the original image. Instead of using the enhanced image as a first or second reader, the enhanced image acts as a joint reader with the user, who can examine the original image while using the enhanced image as assistance.

In one embodiment, the user is able to change enhancement parameters of the enhanced image while viewing the original and enhanced image. In this way, the user is able to adjust the parameters to the most suitable settings for the image while observing the effect on the enhanced image and comparing this with his or her own analysis of the original image. The adjusted parameters may then be applied to other parts of the original image so as to provide a more accurate analysis of those parts.

### Brief Description of the Drawings

Specific embodiments of the present invention will now be illustrated with reference to the accompanying drawings, in which:
Figure 1 is a schematic diagram showing a CT scanner and a remote computer for processing image data from the scanner and operating a user interface;
Figure 2 is a flow chart of the main steps of the method of operation of the user interface in an embodiment of the invention;
Figure 3 is a screenshot of the user interface, with a sphericity enhancement filter applied;
Figure 4 shows a filter window of the user interface with the sphericity enhancement filter selected;
Figure 5 is a screenshot of the user interface, with an edge enhancement filter applied;
Figure 6 shows the filter window with a standard noise removal filter selected;
Figure 7 shows the filter window with an advanced noise removal filter selected; and
Figure 8 is a screenshot of the user interface in a second embodiment, for use with colon scan images.

### Detailed Description of the Embodiments

### Scan Image

A CT image may include one or more slices obtained from a human or animal patient using a CT scanner. Each slice is a 2-dimensional digital grey-scale image of the x-ray absorption of the scanned area. The properties of the slice may depend on the CT scanner used; for example, a high-resolution multi-slice CT scanner may produce images with a resolution of 0.5-0.6 mm/pixel in the x and y directions (i.e. in the plane of the slice). Each pixel may have 32-bit greyscale resolution. The intensity value of each pixel is normally expressed in Hounsfield units (HU). Sequential slices may be separated by a constant distance along the z direction (i.e. the scan separation axis); for example, by a distance of between 0.75-2.5 mm. Hence, the scan image may be a two-dimensional (2D) or three-dimensional (3D) grey scale image, with an overall size depending on the area and number of slices scanned.

The present invention is not restricted to any specific scanning technique, and is applicable to electron beam computed tomography (EBCT), multi-detector or spiral scans or any technique which produces as output a 2D or 3D image representing X-ray absorption.

### Computer System

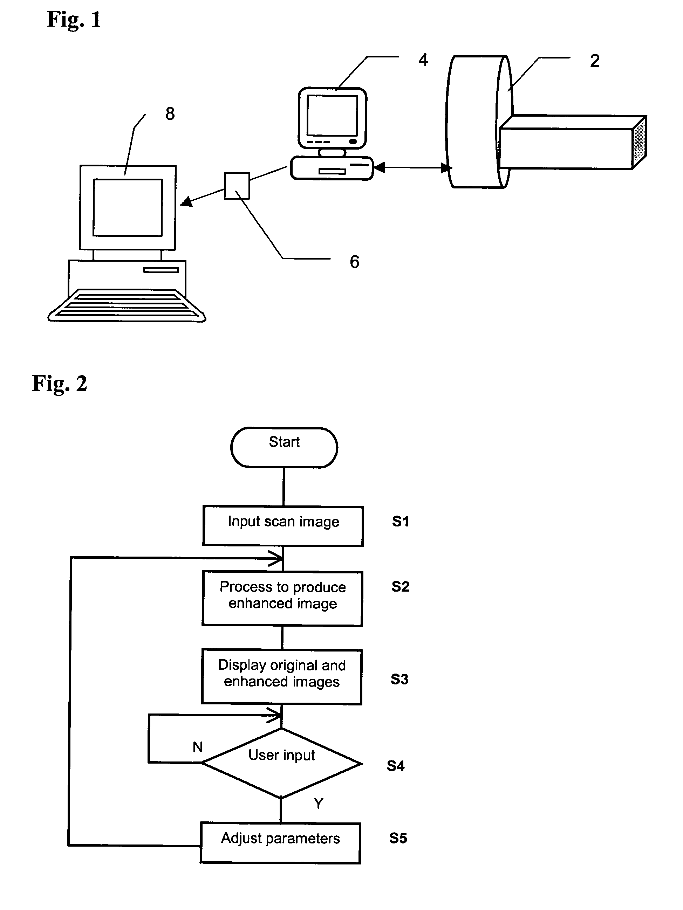

As shown in Figure 1, the scan image is created by a computer 4 which receives scan data from a scanner 2 and constructs the scan image. The scan image is saved as an electronic file or a series of files which are stored on a storage medium 6, such as a fixed or removable disc. The scan image may be stored in a standard format, such as DIRCOM 3.0. The scan image may be processed by the computer 4, or the scan image may be transferred to another computer 8 which runs software for processing and displaying images as described below. The software may be stored on a carrier, such as a removable disc, or downloaded over a network.

### User Interface Flowchart

A flowchart of a method of operation of a user interface, implemented by software in an embodiment of the invention, is shown in Figure 2. An original scan image is provided as input (S1) and is processed to provide an enhanced scan image (S2). A representation of the original scan image and the enhanced scan image are displayed (S3), either side by side or alternately, together with an indication of the parameter values used for the enhancement. If a user input is received (S4) to adjust the parameter values, the values are adjusted (S5) and the enhanced scan image is produced again (S2) using the adjusted parameters.

The original and enhanced images may be displayed side by side, or may be displayed alternately at the same position and with the same size and scale; for example, the image enhancement may be alternately switched on and off. The switching between original and enhanced images may occur every time the user presses a specific key, or clicks on a button on-screen.

### Lung Scan Embodiment

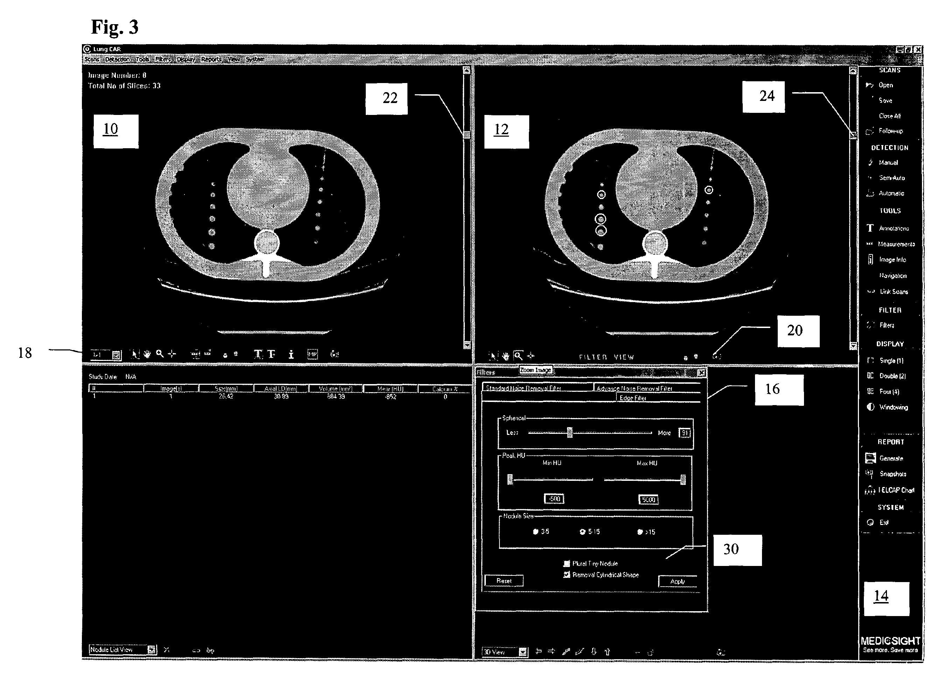

Figure 3 shows a screenshot of the user interface in a first embodiment, displaying a two-dimensional slice of a scan of a lung phantom (i.e. a model approximating the lung and containing objects with known properties, used for testing and calibration).

The user interface is shown in a window comprising an original image pane 10, and an enhanced image pane 12. The original image pane 10 displays a slice of the scan image. A first toolbar 18 is located at the bottom of the original image pane 10, containing buttons which allow the user to manipulate the original image, for example by zooming, panning and rotating. The original image pane 10 also includes a first scroll bar 22 which allows the user to move forward and backward between slices.

The enhanced image pane 12 displays a version of the original image processed by one or more filters so as to highlight features of the original image. In this example, a sphericity enhancement filter is applied, and objects satisfying the sphericity filter are circled. A second toolbar 20 in the enhanced image pane 12 contains buttons which allow the user to manipulate the enhanced image, for example by zooming, panning and rotating. The enhanced image pane 12 also includes a second scroll bar 24 which allows the user to move forward and backward between slices.

The first and second toolbars 18, 20 may be linked so that an image manipulation performed by either toolbar has a corresponding or equivalent effect on both the original and the enhanced image. For example, magnifying the original scan image using the first toolbar 18 magnifies the enhanced scan image in the enhanced image pane 12 by the same amount. In another example, magnifying the enhanced scan image using the second toolbar 20 magnifies the original scan image in original image pane 10 by the same amount. In this way, a side-by-side comparison may be made between the same view of the original and enhanced images.

The first and second scroll bars 22, 24 may also be linked so that moving either scrollbar has a corresponding or equal effect on both the original scan image and the enhanced scan image. This allows the user to move back and forth in the z-direction while comparing the original and enhanced scan images.

The user interface window includes a right-hand toolbar 14 which displays various options selectable by the user. A 'Display' option allows the user to control how many panes are displayed: single, two, four or window, in which the panes are displayed as separate windows. In this example, only two panes are used for displaying scan images, while the third and fourth pane are assigned a data display and 3D nodule display function respectively.

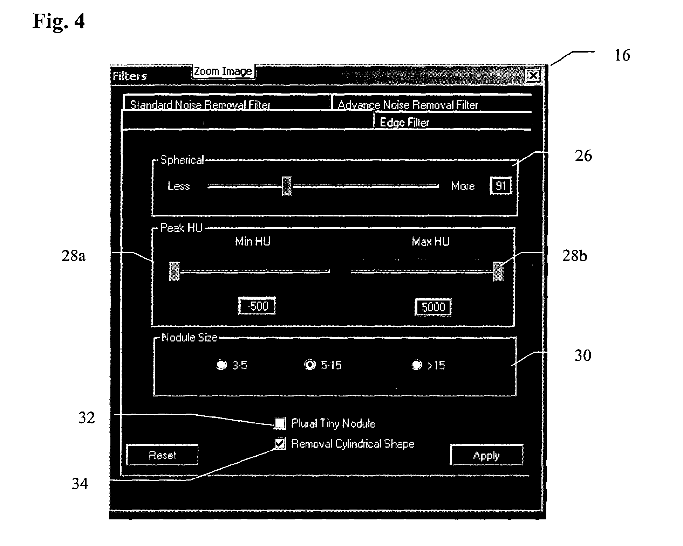

The type and parameters of the filter are controlled by the user by means of a filter window 16, which is shown in more detail in Figure 4. In this example, the user can select any one of a sphericity enhancement filter, an edge filter, a standard noise removal filter and an enhanced noise removal filter, by selecting the corresponding tab. The user then sets the parameter values of the filter, and clicks a button to apply the filter to update the enhanced image.

### Sphericity Enhancement Filter

In this example, the sphericity enhancement filter tab includes a sphericity slider 26 allowing the user to set the minimum level of sphericity for objects passed by the filter, a minimum intensity slider 28a and a maximum intensity slider 28b allowing the user to select the minimum and maximum peak intensity respectively within an object to be passed by the filter, and a nodule size selector 30 allowing the user to select one of a plurality of different size ranges of objects to be passed by the filter. The user can also select a first option 32 to detect plural tiny nodules, and a second option 34 to remove cylindrical shapes. The latter provides the user with the ability to avoid enhancement of spherical shapes that are within cylindrical structures, such as blood vessels.

The sphericity enhancement filter may operate by analysing each volume element (voxel) within the scan image and comparing with surrounding voxels of similar intensity to derive the three-dimensional curvature of a surface of equal intensity. Surfaces having a sphericity above the level selected by the user are identified as belonging to spherical objects. Voxels contained within those surfaces are then grouped together as parts of the same object. Once all such objects have been identified, those having a maximum intensity between the minimum and maximum selected by the user, and a size within the range selected by the user, are highlighted by the filter.

Where a spherical object passed by the filter occupies multiple consecutive slices, the object may be highlighted only on the slice which contains the greatest area of the object.

### Edge Enhancement Filter

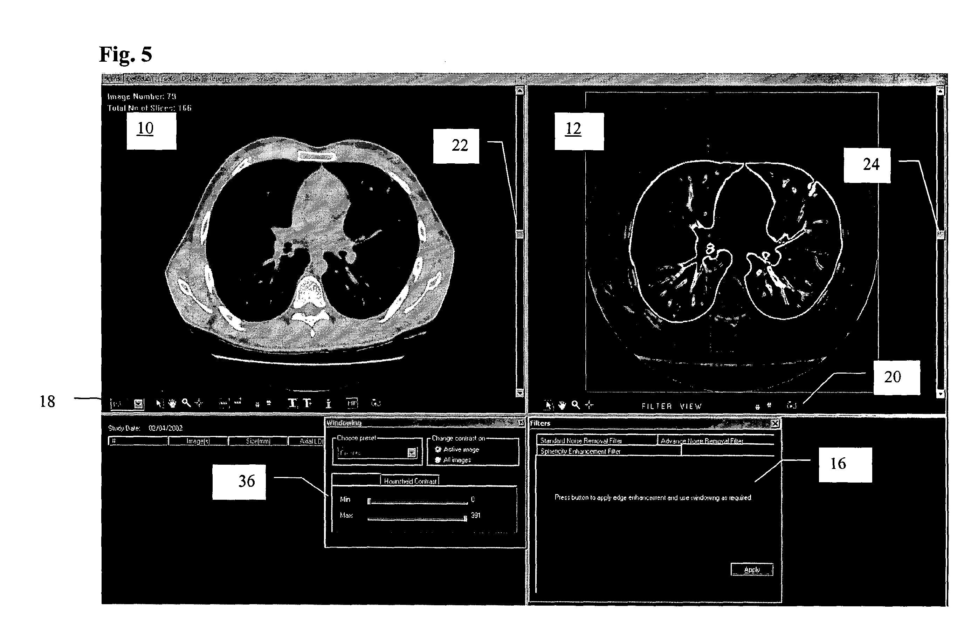

Figure 5 shows the embodiment when applied to another scan image, with the edge enhancements filter selected and applied. The edge enhancement is applied to the lung parenchyma area only, and is advantageous in identifying signs of interstitial disease such as reticulation.

When the edge enhancement filter is selected, a contrast setting window 36 is displayed, allowing the user to vary contrast parameters of the edge enhancement filter.

### Standard Noise Removal Filter

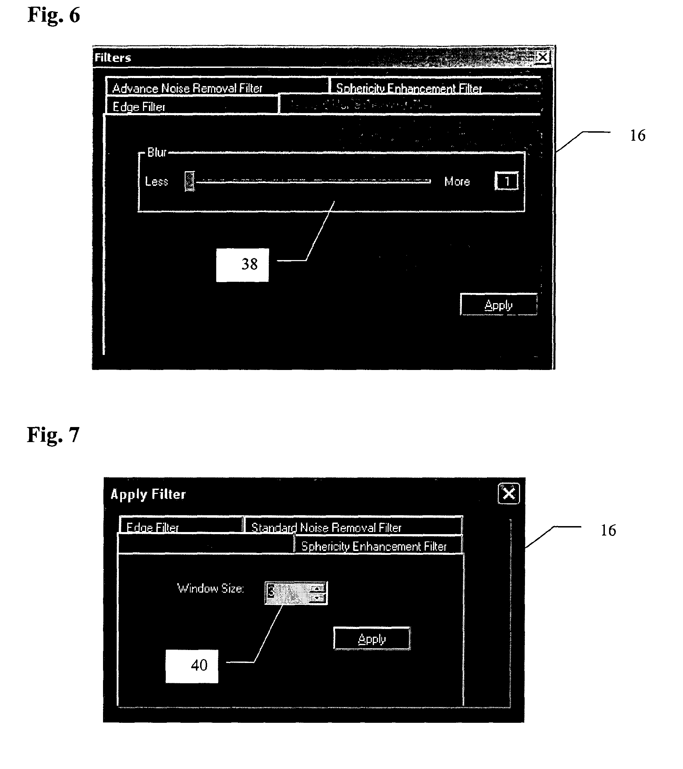

Figure 6 shows the filter window 16 when the standard noise removal filter is selected. The filter window displays a blur slider 38 by which the user can vary the degree of noise smoothing. This filter is advantageous for reading low-dose multi-slice spiral CT (MSCT) studies, where radiation dose reduction is achieved at the expense of increased background noise. The standard noise removal filter smoothes the background noise to the degree set by the user.

### Advanced Noise Removal Filter

Figure 7 shows the filter window 16 when the advanced noise removal filter is selected. The filter window 16 displays a window size selector 40 which sets the window size used by the advanced noise reduction technique. This technique results in less apparent blurring than the standard technique, but is more time consuming.

### Colon Scan Embodiment

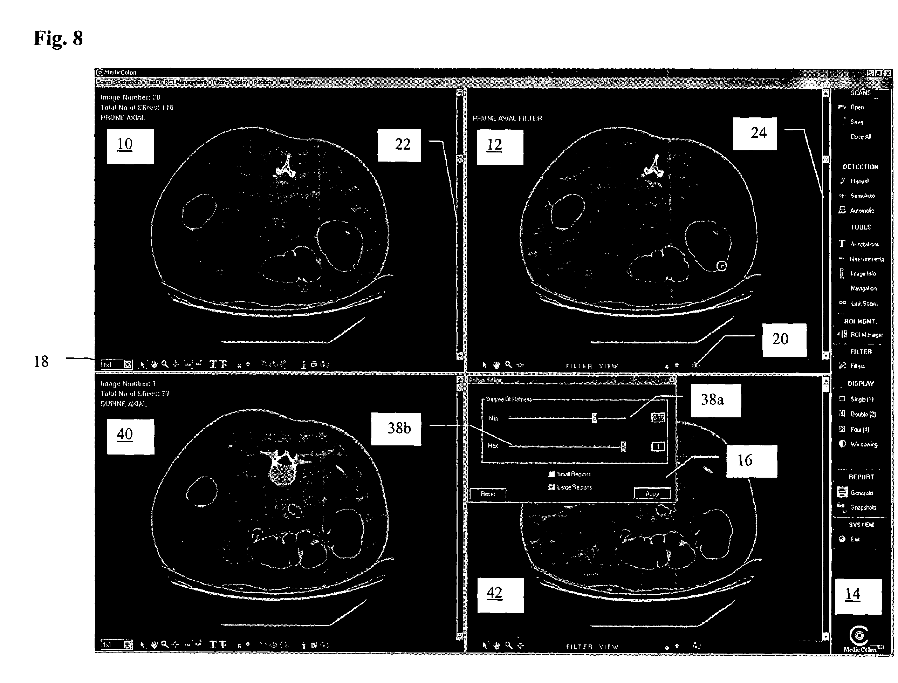

Figure 8 shows a screen display of a user interface in a second embodiment, for use with scan images of the colon. This embodiment differs from the lung scan embodiment in the type of filters available; a single polyp enhancement filter is available rather than the choice of filters used in the lung scan embodiment. However, any of the filters available in the lung scan embodiment may be used with the second embodiment. Similar parts are shown with the same reference numerals, and their description is not repeated.

The filter window 16 displays settings for the polyp enhancement filter which highlights raised objects with a spherical element, but does not highlight objects that are elongated and likely to be folds. The filter window includes minimum and maximum flatness sliders 38a, 38b which allow the user to select the degree of flatness of objects to be highlighted.

In this example, the third and fourth panes comprise a second original image pane 40 and a second enhanced image pane 42, displaying a supine axial view of the scan image while the original image pane 10 and enhanced image pane 12 display a prone axial view. Manipulation of the second original image pane 40 and a second enhanced image pane 42 displays is also linked so that changes made in one pane are echoed in the other.

### Single View Embodiment

As an alternative to the side-by-side views described above, the user may select a display mode in which only a single scan image view is displayed in the user interface window, and the enhancement selected by the filter window can be switched on and off by the user, for example by toggling a button in the filter window. Image display parameters are kept unchanged when switching between the original and the enhanced image, so that the user can easily compare the enhancement and the original image.

### Alternative Embodiments

The embodiments described above are illustrative of rather than limiting to the present invention. Alternative embodiments apparent on reading the above description may nevertheless fall within the scope of the invention

## Claims

1. A computer-implemented method of displaying a computed tomography (CT) scan image, comprising:
a) processing (S2) an original scan image to generate an enhanced scan image in which one or more features indicative of an abnormality in the original scan image are enhanced and at least one other feature of the original scan image is obscured; and **characterised by**:
b) displaying (S3) the original scan image and the enhanced scan image so as to enable a visual comparison therebetween.

2. The method of claim 1, wherein the original scan image (10) and the enhanced scan image (12) are displayed simultaneously.

3. The method of claim 1, wherein the original scan image and the enhanced scan image are displayed alternately.

4. The method of claim 3, wherein the display alternates between the original scan image and the enhanced scan image in response to a user input.

5. The method of claim 3 or claim 4, wherein the original scan image and the enhanced scan image are displayed alternately in substantially the same position.

6. The method of any preceding claim, including modifying (S5) a displayed representation (10) of the original scan image and a displayed representation (12) of the enhanced scan image, in response to a user input (S4).

7. The method of claim 6, wherein the displayed representation (10) of the original scan image is a slice of the original scan image and the displayed representation (12) of the enhanced scan image is a corresponding slice of the enhanced scan image, and the displayed slice of the original scan image and the corresponding slice of the enhanced scan image are modified in response to the user input.

8. The method of any preceding claim, wherein the processing step (S2) is performed according to one or more user-defined parameters, the method including receiving a user input for modifying one or more said parameters, and performing the processing and displaying steps using said modified one or more parameters.

9. The method of any preceding claim, wherein the processing step is performed using a selected one of a plurality of filters, the method including receiving a user input for selecting one of said filters, and performing the processing and displaying steps using the selected filter.

10. The method of any preceding claim, wherein the original scan image is a scan image of a lung, and the original scan image is processed (S2) to enhance spherical objects in the enhanced scan image.

11. The method of any preceding claim, wherein the original scan image is a scan image of a lung, and the original scan image is processed (S2) to enhance edges in the enhanced scan image.

12. The method of any one of claims 1 to 9, wherein the original scan image is a scan image of a colon, and the original scan image is processed (S2) to enhance spherical objects raised from the colon wall in the enhanced scan image.

13. A computer program arranged to perform the method of any preceding claim.

14. A computer program product comprising the computer program of claim 13, recorded on a carrier.

15. Apparatus (8) for displaying a computed tomography (CT) scan image, comprising:
a) means arranged to process an original scan image to generate an enhanced scan image in which one or more features indicative of an abnormality in the original scan image are enhanced and at least one other feature of the original scan image is obscured; and
b) means arranged to display the original scan image and the enhanced scan image so as to enable a visual comparison therebetween.
